# EUROPEAN PATENT SPECIFICATION

(11) **EP 4 324 503 B1**
(45) Date of publication and mention of the grant of the patent: **05.11.2025**
(21) Application number: 23764552.8
(22) Date of filing: 02.06.2023
(51) Int. Cl.: A61M 15/00, A61M 11/02

(54) **INHALER**
INHALATOR
INHALATEUR

(30) Priority: 01.07.2022 KR 20220081053
(43) Date of publication of application: 21.02.2024
(73) Proprietor: KT & G Corporation, Daejeon 34337 (KR)
(72) Inventor: KIM, Tae Heon, Daejeon 34128 (KR); KIM, Jae Hyun, Daejeon 34128 (KR); LEE, Mi Jeong, Daejeon 34128 (KR); JEONG, Minseok, Daejeon 34128 (KR); JUNG, Yongmi, Daejeon 34128 (KR); JEOUNG, Eunmi, Daejeon 34128 (KR); CHUNG, Tae Young, Daejeon 34128 (KR); HAN, Seung Kyu, Daejeon 34128 (KR); KIM, Dong Sung, Daejeon 34128 (KR)
(74) Representative: Ter Meer Steinmeister & Partner
(86) International application number: PCT/KR2023/007575
(87) International publication number: WO 2024/005389

(56) References cited:
- EP-A1- 4 302 809
- WO-A1-2020/007413
- WO-A1-99/49916
- JP-A- 2006 505 374
- JP-A- 2020 151 022
- JP-B2- 2 845 887
- US-A- 4 576 157
- US-A1- 2007 074 718

## Description

### Technical Field

Disclosed is an inhaler.

### Background Art

In general, an inhaler is a device used to inhale a composition such as medication through the oral cavity or nasal cavity as a liquid or gas in the process of inhalation. Such an inhaler may include a container accommodating an inhalable composition, and the composition may be sprayed from the container through a thin tube to the oral cavity or nasal cavity through an intake to be inhaled by a user.

The above description has been possessed or acquired by the inventor(s) in the course of conceiving the present disclosure and is not necessarily an art publicly known before the present application is filed.
Prior Document: Korean Patent Gazette No. 10-2021229 (Published on September 11, 2019) EP 4 302 809 A1 concerning an inhaler represents prior art under Art. 54 (3) EPC. WO 2020/007413 A1, US 2007/074718 A1, JP 2006 505374 A, US 4 576 1 57 A, and JP 2006 505374 A respectively relate to aerosol dispersion devices with a pressurized container and the possibility to actuate a lever or the container to release a consumable substance via a mouthpiece.

### Disclosure of the Invention

### Technical Goals

An object according to an embodiment is to provide an inhaler, in which an aerosol is easily formed, a spraying amount is easily adjusted, refilling and inhalation are convenient with the use of a canister integration type, and a canister is easily replaced.

An object according to an embodiment is to provide an inhaler obtained by solving hygiene issues by applying a mouthpiece that is easily detached and cleaned, and considering various user conveniences such as a filling lever, a relief valve, a counter, and the like.

The technical tasks obtainable from the present disclosure are non-limited by the above-mentioned technical tasks. And, other unmentioned technical tasks can be clearly understood from the following description by those having ordinary skill in the technical field to which the present disclosure pertains.

### Technical Solutions

The invention is defined by the appended claims. An inhaler according to an embodiment for achieving the above objects includes: a housing having one surface, the other surface opposite to the one surface, and a plurality of side surfaces connecting the one surface and the other surface; a mouthpiece which is disposed on the one surface of the housing; a reservoir which communicates with the mouthpiece and stores an inhalable composition; and a canister which accommodates the inhalable composition to be stored in the reservoir and is connected to the reservoir to be opened or closed, and the canister is detachably coupled to the reservoir in the housing.

According to an aspect, the inhaler may further include: a nozzle which extends from the mouthpiece to the reservoir; and a needle valve which is movably disposed inside the nozzle. When a suction force is not applied to the mouthpiece, the needle valve may be maintained in a first state in which the nozzle is closed, and when a suction force is applied through the mouthpiece, the needle valve may be switched into a second state in which the nozzle is opened.

According to an aspect, the inhaler may further include a sealing member which is fixedly provided on one side of the nozzle. In the first state, a first gap may be formed between a front end of the needle valve and the nozzle and a lower end of the needle valve may come into contact with the sealing member, and in the second state, a second gap may be formed between the front end of the needle valve and the sealing member.

According to the invention, the inhaler further includes a lever having one side forming a portion of the housing and the other side being movable between a bottom surface of the canister and the other surface of the housing. When the lever is pressed, the one side of the lever may move in a first direction toward the canister and the other side of the lever may press the canister to move in a second direction toward the reservoir.

According to an aspect, the inhaler includes a relief valve which is provided on the reservoir to be spaced apart from the canister and operates so that the reservoir is opened or closed to the outside. The lever may control the canister to be opened after the relief valve is pre-opened.

According to an aspect, the inhaler may further include a counter which is rotatably disposed between the canister and the lever. The counter may be rotated at a predetermined angle according to the operation of the lever to notify a remaining amount of the inhalable composition in the canister.

According to an aspect, the inhaler may further include: a cover having one side rotatably coupled to the housing and the other side detachably coupled to a position corresponding to the bottom surface of the canister; and a cover lock which is disposed adjacent to the one side of the cover and to be slidable to a locking position restraining rotation of the cover or an unlocking position allowing the rotation of the cover. The cover lock may open the relief valve in the unlocking position.

### Effects

According to the inhaler according to an embodiment, there are effects that it is easy to form an aerosol and adjust a spraying amount, refilling and inhalation are convenient with the use of a canister, and a canister is easily replaced.

According to the inhaler according to an embodiment, there are effects that the hygiene issues are solved by applying a mouthpiece that is easily detached and cleaned, and various user conveniences such as a filling lever, a relief valve, a counter, and the like are provided.

The effects of the inhaler are not limited to the above-mentioned effects, and other unmentioned effects can be clearly understood from the following description by one of ordinary skill in the art to which the present disclosure pertains.

### Brief Description of Drawings

FIG. 1 is a perspective view of an inhaler according to an embodiment.
FIG. 2 is a cross-sectional view of an inhaler according to an embodiment.
FIG. 3 is a cross-sectional view of an inhaler according to an embodiment.
FIG. 4 illustrates an inhaler in a first state and a second state
FIG. 5 is a cross-sectional view of an inhaler according to an embodiment in a state where a lever is not pressed.
FIG. 6 is a cross-sectional view of an inhaler according to an embodiment in a state where a lever is pressed.
FIG. 7 illustrates a relief valve that operates in association with a lever.
FIG. 8 is a perspective view of a rear side of an inhaler according to an embodiment.
FIG. 9 illustrates a counter that operates in association with a lever.
FIG. 10 illustrates a counter that rotates when a lever is pressed.
FIG. 11 illustrates a counter that returns to an original position when a canister is removed.
FIG. 12 illustrates a cover and a cover lock of an inhaler according to an embodiment.
FIG. 13 illustrates an inhaler 10 according to an embodiment in which a cover lock is in a locked position.
FIG. 14 illustrates an inhaler according to an embodiment in which a cover lock is in an unlocked position.

The accompanying drawings illustrate desired embodiments of the present disclosure and are provided together with the detailed description for better understanding of the technical idea of the present disclosure.

### Best Mode for Carrying Out the Invention

Hereinafter, embodiments will be described in detail with reference to the illustrative drawings. Regarding the reference numerals assigned to the components in the drawings, it should be noted that the same components will be designated by the same reference numerals, wherever possible, even though they are shown in different drawings. Further, in the following description of the present embodiments, a detailed description of publicly known configurations or functions incorporated herein will be omitted when it is determined that the detailed description obscures the subject matters of the present embodiments.

In addition, the terms first, second, A, B, (a), and (b) may be used to describe constituent elements of the embodiments. These terms are used only for the purpose of discriminating one constituent element from another constituent element, and the nature, the sequences, or the orders of the constituent elements are not limited by the terms. When one constituent element is described as being "connected", "coupled", or "attached" to another constituent element, it should be understood that one constituent element can be connected or attached directly to another constituent element, and an intervening constituent element can also be "connected", "coupled", or "attached" to the constituent elements.

The same name may be used to describe an element included in the embodiments described above and an element having a common function. Unless otherwise mentioned, the descriptions on the embodiments may be applicable to the following embodiments and thus, duplicated descriptions will be omitted for conciseness.

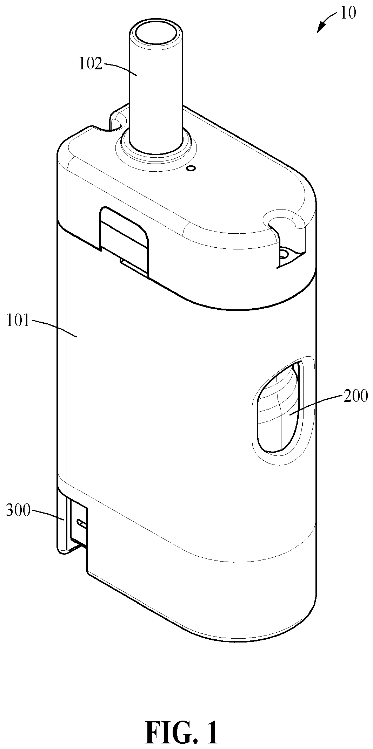

FIG. 1 is a perspective view of an inhaler 10 according to an embodiment.

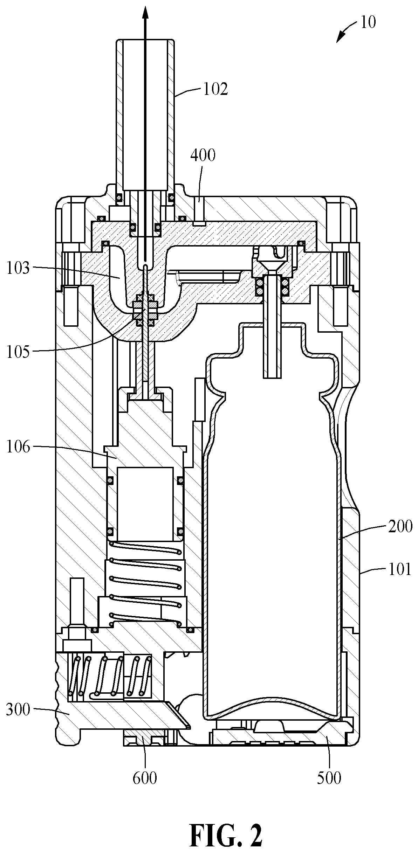

FIG. 2 is a cross-sectional view of the inhaler 10 according to an embodiment.

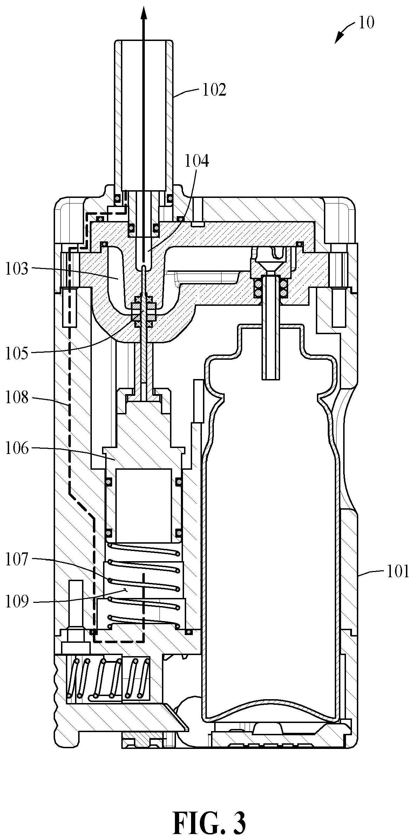

FIG. 3 is a cross-sectional view of the inhaler 10 according to an embodiment.

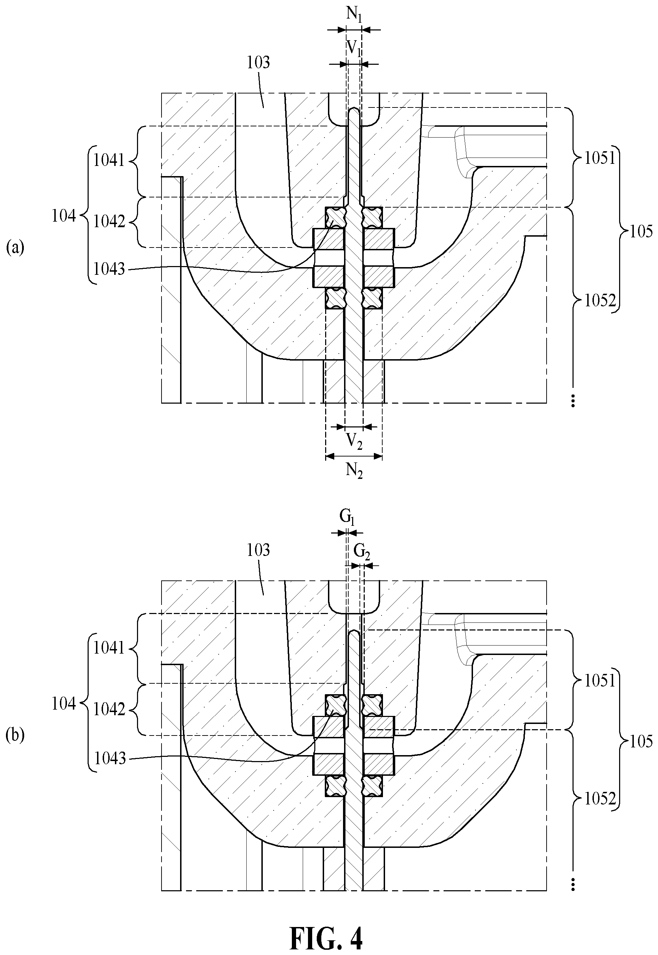

FIG. 4 illustrates the inhaler 10 in a first state and a second state.

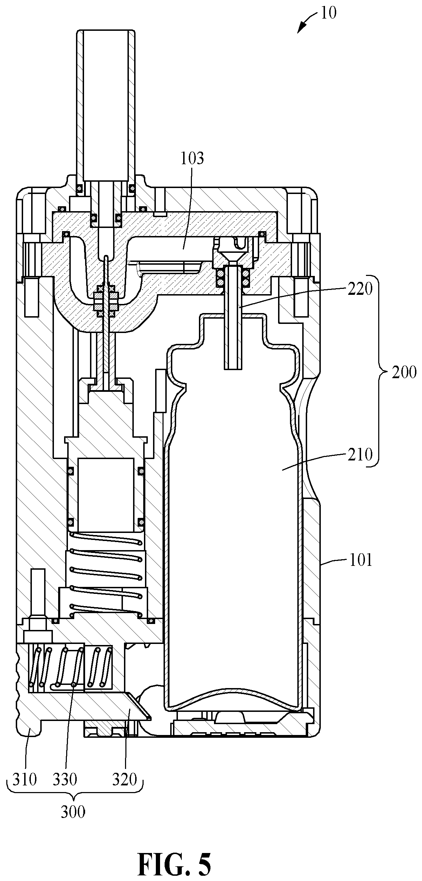

FIG. 5 is a cross-sectional view of the inhaler 10 according to an embodiment in a state where a lever 300 is not pressed.

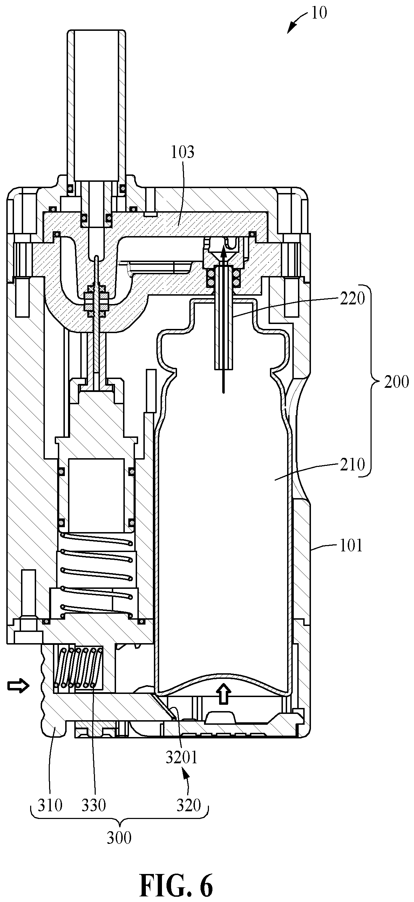

FIG. 6 is a cross-sectional view of the inhaler 10 according to an embodiment in a state where the lever 300 is pressed.

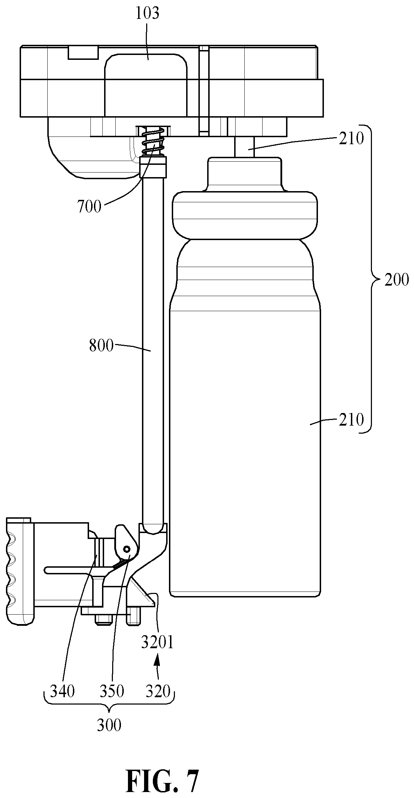

FIG. 7 illustrates a relief valve 700 that operates in association with the lever 300.

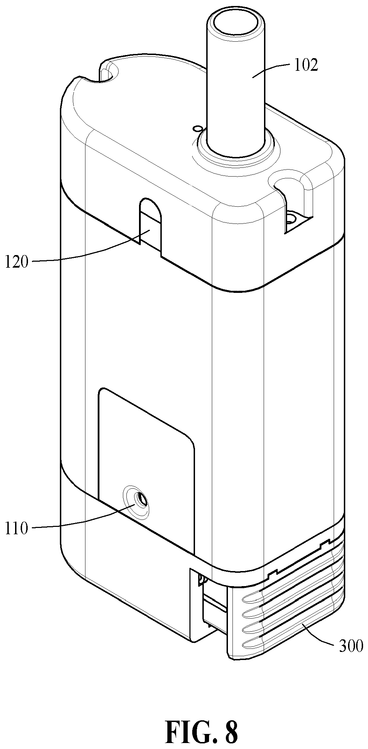

FIG. 8 is a perspective view of a rear side of the inhaler 10 according to an embodiment.

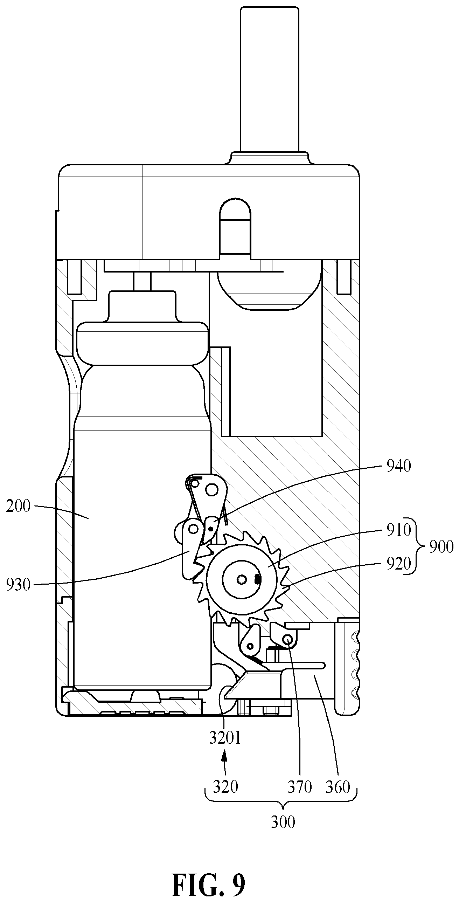

FIG. 9 illustrates a counter 900 that operates in association with the lever 300.

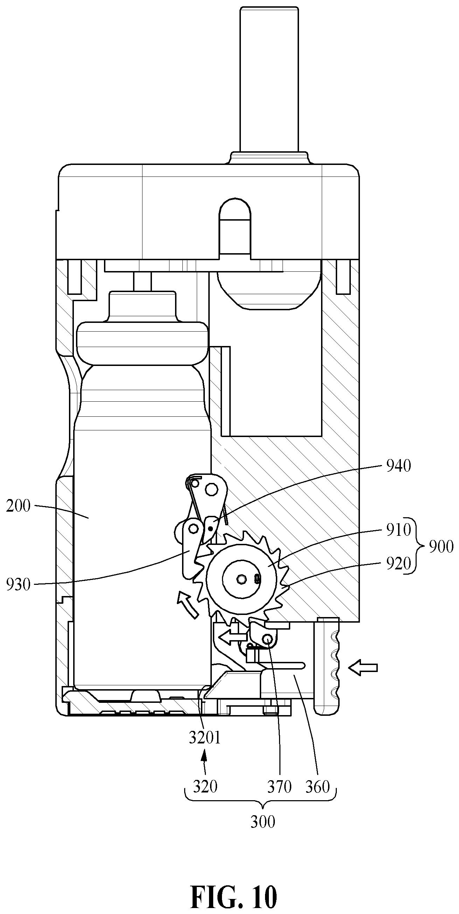

FIG. 10 illustrates the counter 900 that rotates when the lever 300 is pressed.

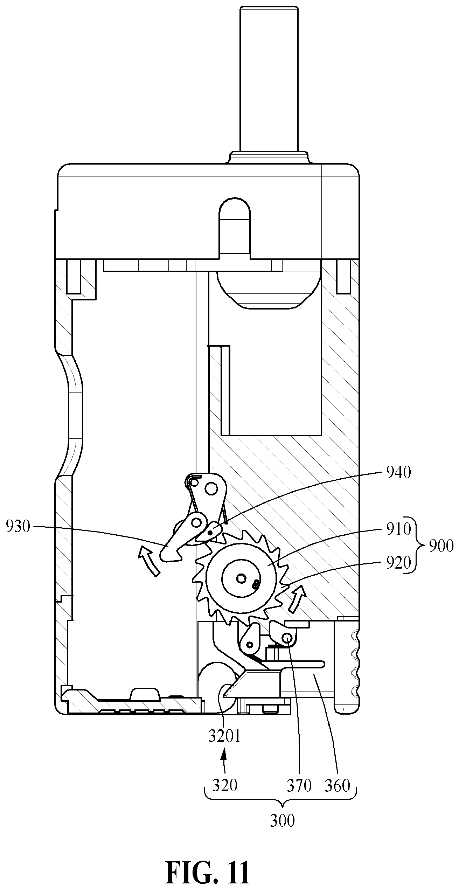

FIG. 11 illustrates the counter 900 that returns to an original position when a canister 200 is removed.

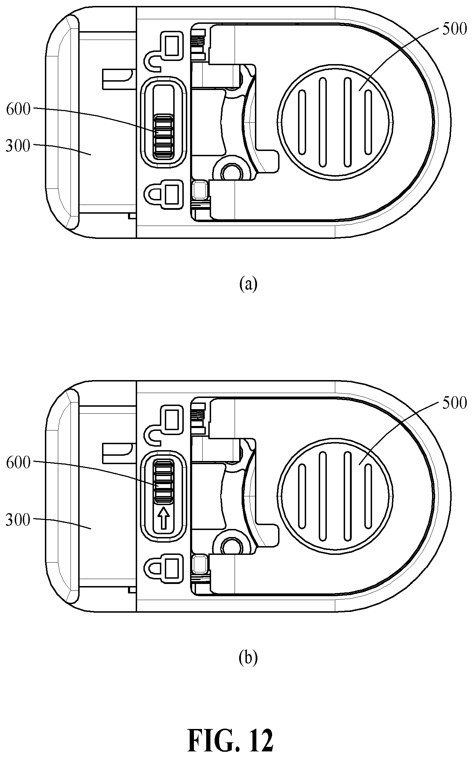

FIG. 12 illustrates a cover 500 and a cover lock 600 of the inhaler 10 according to an embodiment.

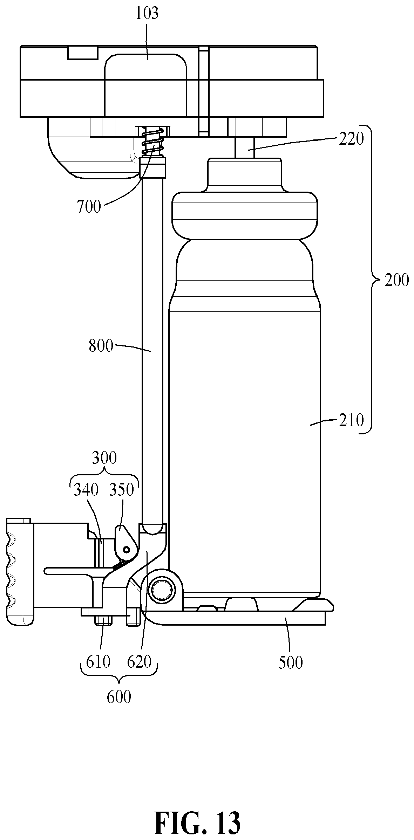

FIG. 13 illustrates the inhaler 10 according to an embodiment in which the cover lock 600 is in a locked position.

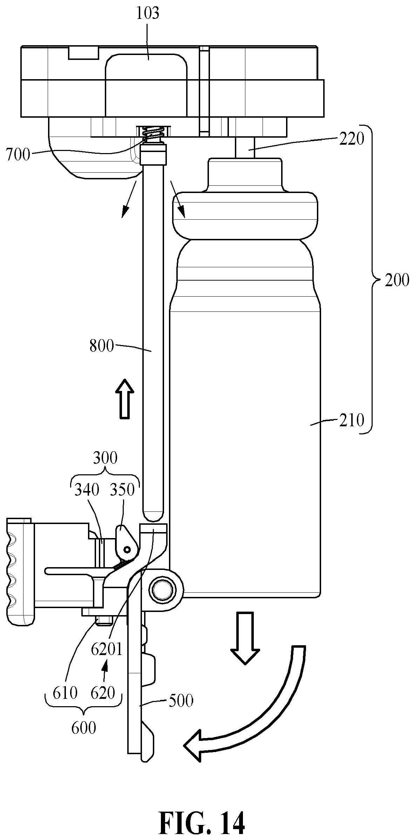

FIG. 14 illustrates the inhaler 10 according to an embodiment in which the cover lock 600 is in an unlocked position.

Referring to FIG. 1, the inhaler 10 according to an embodiment may include a housing 101 and a mouthpiece 102.

The housing 101 may include a first surface formed on one surface, a second surface opposite to the first surface, and a plurality of side surfaces connecting the first surface and the second surface. The first surface of the housing 101 may be, for example, a surface located on the top of the housing 101, and the second surface may be, for example, a bottom surface of the housing 101. Hereinafter, a direction from the second surface to the first surface is defined as a first direction, and a direction from the first surface to the second surface is defined as a second direction.

The mouthpiece 102 may be disposed on the first surface of the housing 101. A user may inhale an inhalable composition accommodated in the inhaler 10 through the mouthpiece 102. At this time, the user may inhale the composition, for example, in the form of an aerosol or in the form of a powder. Hereinafter, the inhaler 10 according to an embodiment will be described using the inhaler 10 that sprays an inhalable composition in the form of an aerosol as an example.

Referring to FIGS. 1 and 2, a reservoir 103 may be disposed inside the housing 101. Since one side of the canister 200 is connected to the reservoir 103, the inhalable composition accommodated in the canister 200 may be moved to and stored in the reservoir 103. In addition, as the user applies a suction force through the mouthpiece 102, the inhalable composition stored in the reservoir 103 may be discharged through the mouthpiece 102 in the form of an aerosol, and accordingly, the inhalable composition stored in the reservoir 103 may be gradually consumed. The composition stored in the reservoir 103 may be sprayed in the form of an aerosol as the user applies a suction force to the mouthpiece 102. At this time, an inhalation interlocking valve 105 that opens and closes the reservoir 103 by the suction force may operate, and the opening and closing operation of the inhalation interlocking valve 105 may be controlled by a piston 106.

The canister 200 may be mounted inside the housing 101. In this case, the canister 200 may be mounted interchangeably inside the housing 101. In addition, the canister 200 may accommodate an inhalable composition therein. A certain amount of the composition of the canister 200 may be filled in the reservoir 103.

The lever 300 may be pressed by the user to fill the inside of the reservoir 103 with the composition accommodated in the canister 200. The lever 300 may form one side surface of the housing 101 and may be positioned adjacent to the second surface. When the user presses the lever 300, the lever 300 may push up a bottom surface of the canister 200 so that an injection hole 220 of the canister 200 communicates with the reservoir 103, and the composition may move from the canister 200 to the reservoir 103 through the injection hole 220.

Hereinafter, a direction from one side surface of the housing 101 where the lever 300 is located to the canister 200 is defined as a first direction, and a direction from the second surface of the housing 101 to the first surface is defined as a second direction.

Meanwhile, the relief valve 700 may be provided on the reservoir 103, and a relief vent hole 400 may be provided on the first surface of the housing 101. The relief valve 700 may be interlocked with the lever 300 through a relief bar 800, a first protrusion member 350, or the like, and the relief valve 700 may operate to open before the lever 300 pushes the canister 200 up. Accordingly, a residual gas in the reservoir 103 may be discharged before the reservoir 103 is filled with the inhalable composition.

Referring to FIG. 8, in the inhaler 10 according to an embodiment, the user may visually identify a remaining amount of the composition stored in the reservoir 103 through a viewing window 120 provided on the housing 101. In addition, the inhaler 10 according to an embodiment may include the counter 900 that counts the number of times of filling in connection with a vertical movement of the canister 200 during the filling of the reservoir 103, and a display window 110 that displays a remaining amount of the composition in the canister 200.

The inhaler 10 according to an embodiment may further include a cover 500 and a cover lock 600 for preventing the canister 200 from being separated.

In the inhaler 10 described above, a needle valve method developed to solve problems that occur when an aerosol is sprayed into the user's nasal cavity or oral cavity is applied to the inside of the housing 101, and this needle valve method will be described in detail below with reference to FIGS. 3 and 4.

In general, when a pinch valve method is applied to an inhaler, a problem of wetting the inside of the user's oral cavity may occur due to too large particles of the aerosol sprayed through a nozzle having a circular cross-sectional area. In addition, since a tip of an injection nozzle is positioned inside the oral cavity, a problem in that the aerosol hits the oral cavity hard during the spraying may occur. In order to solve such problems, the needle valve method is applied in the present disclosure.

Referring to FIG. 3, the inhaler 10 according to an embodiment may further include the reservoir 103, a nozzle 104, and a needle valve 105.

The reservoir 103 may be disposed inside the housing 101. The reservoir 103 may store an inhalable composition.

The nozzle 104 may allow the mouthpiece 102 to communicate with the reservoir 103. The nozzle 104 may be provided as a tube extending from the mouthpiece 102 to the reservoir 103.

The needle valve 105 may be movably disposed inside the nozzle 104. For example, the needle valve 105 may move up and down inside the nozzle 104. The needle valve 105 may open or close the nozzle 104 depending on its position within the nozzle 104. That is, the needle valve 105 may open the nozzle 104 so that the mouthpiece 102 and the reservoir 103 communicate with each other, or may close the nozzle 104 so that the mouthpiece 102 and the reservoir 103 are isolated from each other.

In the needle valve method using the needle valve 105, as the needle-shaped valve 105 is positioned in the middle of the nozzle 104, a cross-sectional area for spraying an aerosol may maintain a donut shape. In addition, finer particles may be formed by spraying the aerosol from a narrower section compared to the same injection area, and a phenomenon of hitting or wetting the inside of the oral cavity may be improved, because the nozzle 104 for spraying an aerosol is positioned at a lower tip of the mouthpiece 102 which is far from the oral cavity.

The nozzle 104 may be switched to one of a first state or a second state by the needle valve 105. The first state is a state in which the nozzle 104 is closed, and the second state is a state in which the nozzle 104 is open. In the first state, the mouthpiece 102 and the reservoir 103 may be isolated from each other. In the second state, the mouthpiece 102 and the reservoir 103 may communicate with each other. That is, in the second state, the inhalable composition stored in the reservoir 103 may be discharged to the mouthpiece 102 through the nozzle 104 as indicated by an arrow.

In addition, the needle valve 105 may operate in association with suction.

Specifically, when a suction force is not applied to the mouthpiece 102, the needle valve 105 may maintain the nozzle 104 in the first state. When a suction force is applied through the mouthpiece 102, the needle valve 105 may move in the second direction to switch the nozzle 104 into the second state.

Referring back to FIG. 3, the inhaler 10 according to an embodiment may further include the piston 106, a spring 107, a passage 108, and a negative pressure forming portion 109.

The piston 106 may be disposed inside the housing 101, and one end of the needle valve 105 may be coupled to one surface of the piston 106. The piston 106 may reciprocate vertically in a cylinder.

The spring 107 may be provided below the piston 106. At this time, the spring 107 may be provided in a preloaded state.

The passage 108 may be formed inside the housing 101. The passage 108 may allow the mouthpiece 102 to communicate with the piston 106. Specifically, one end of the passage 108 may be connected to one side of the mouthpiece 102, and the other end of the passage 108 may be connected to the bottom of the piston 106. At this time, one end of the passage 108 may be connected to the mouthpiece 102 at a position spaced apart from a position where the nozzle 104 is connected to the mouthpiece 102. The passage 108 may transfer a suction force applied to the mouthpiece 102 to the piston 106.

The negative pressure forming portion 109 may be formed between the piston 106 and one end of the passage 108 connected to the piston 106. The negative pressure forming portion 109 may form a space between the piston 106 and the passage 108. When a suction force is applied to the mouthpiece 102, the suction force transferred through the passage 108 may reach the negative pressure forming portion 109, and the negative pressure forming portion 109 may generate a negative pressure. Accordingly, the negative pressure forming portion 109 may induce the piston 106 to move in the second direction.

As a result, when a suction force is not applied to the mouthpiece 102, the spring 107 may push the piston 106 up in the first direction so that the needle valve 105 and the nozzle 104 are maintained in the first state. On the other hand, when a suction force is applied to the mouthpiece 102, the suction force may be transferred to the negative pressure forming portion 109 through the passage 108, and the piston 106 may overcome the preload of the spring 107 by the negative pressure generated by the negative pressure forming portion 109, and move in the second direction. Accordingly, the needle valve 105 may move in the second direction and the nozzle 104 may be switched into the second state.

Referring to FIG. 4, the nozzle 104 and the needle valve 105 in the first and second states will be described in more detail.

FIG. 4A shows the inhaler 10 in the first state and FIG. 4B shows the inhaler 10 in the second state.

In the first state, the nozzle 104 and the needle valve 105 may come into contact with each other and the mouthpiece 102 and the reservoir 103 may be isolated from each other.

In the second state, the needle valve 105 may move in the second direction so that the nozzle 104 and the needle valve 105 may not come into contact with each other, and the mouthpiece 102 and the reservoir 103 may communicate with each other through the nozzle 104. Accordingly, the inhalable composition in the reservoir 103 may be discharged outside from the reservoir 103 through the mouthpiece 102.

Specifically, the nozzle 104 may include a first nozzle portion 1041 and a second nozzle portion 1042.

The first nozzle portion 1041 may be a portion adjacent to the mouthpiece 102.

The second nozzle portion 1042 may be, for example, a portion located on the bottom of the first nozzle portion 1041 and adjacent to the reservoir 103.

The nozzle 104 may be formed such that the first nozzle portion 1041 has a diameter smaller than that of the second nozzle portion 1042.

The needle valve 105 may include a first valve portion 1051 and a second valve portion 1052.

The first valve portion 1051 may be a portion formed at a front end of the needle valve 105. The first valve portion 1051 may move adjacent to the first nozzle portion 1041 or the second nozzle portion 1042 when the piston 106 moves vertically. For example, in the first state, the first valve portion 1051 may be disposed adjacent to the first nozzle portion 1041. In addition, in the second state, the first valve portion 1051 may move in the second direction to be adjacent to the second nozzle portion 1042.

The second valve portion 1052 may be, for example, a portion formed below the first valve portion 1051. A lower end of the second valve portion 1052 may be coupled to one surface of the piston 106. Accordingly, when the piston 106 vertically reciprocates, the needle valve 105 may vertically reciprocate.

The needle valve 105 may be formed such that the first valve portion 1051 has a diameter smaller than that of the second valve portion 1052.

In addition, the first valve portion 1051 may be formed to have a diameter smaller than that of the first nozzle portion 1041. That is, the first valve portion 1051 may not come into contact with any of the first nozzle portion 1041 and the second nozzle portion 1042 in the first state or the second state.

Meanwhile, the nozzle 104 may further include a sealing member 1043 provided on the second nozzle portion 1042. The sealing member 1043 may be provided as, for example, an O-ring or a Quad-ring formed of an elastic material such as silicon or rubber. An inner diameter of the sealing member 1043 may be larger than the diameter of the first valve portion 1051 and may be equal to or smaller than the diameter of the second valve portion 1052. Also, the inner diameter of the sealing member 1043 may be larger than the diameter of the first nozzle portion 1041.

The second valve portion 1052 may be disposed adjacent to the sealing member 1043 in the first state. That is, in the first state, the second valve portion 1052 may come into contact with the sealing member 1043. Accordingly, the reservoir 103 may be sealed airtightly so that no aerosol leaks through the nozzle 104.

On the other hand, the second valve portion 1052 may move in the second direction with respect to the sealing member 1043 and may not to come into contact with the nozzle 104 in the second state. Accordingly, the inhalable composition stored in the reservoir 103 may be discharged to the mouthpiece 102 through the nozzle 104.

Referring to FIG. 4B, a gap formed between the first nozzle portion 1041 and the first valve portion 1051 is defined as a first gap G1, and a gap formed between the sealing member 1043 and the first valve portion 1051 is defined as a second gap G2. Due to the structure of the nozzle 104 and the needle valve 105 described above, the first gap G1 may be formed to be smaller than the second gap G2.

The first gap G1 may be formed in the first state or the second state. The second gap G2 is formed when the first valve portion 1051 is disposed adjacent to the sealing member 1043, and therefore, the second gap G2 may be formed in the second state in which the needle valve 105 is moved in the second direction.

The second gap G2 may allow the movement of the inhalable composition discharged from the reservoir 103. The first gap G1 may control a particle size of an aerosol which has passed through the second gap G2. That is, since the first gap G1 is smaller than the second gap G2, the first gap G1 may allow only the movement of an aerosol with fine particles from the aerosol. As a result, only an aerosol having a size that is able to pass through the first gap G1 may move to the mouthpiece 102 to be discharged to the user.

That is, when the nozzle 104 is opened by a suction force, the aerosol is finally sprayed to the outside through the first gap G1, but the initial discharge from the reservoir 103 may be performed through the second gap G2 formed between the sealing member 1043 and the first valve portion 1051.

That is, since both the nozzle 104 and the needle valve 105 are formed straight, the first gap G1 is constantly formed regardless of the opening and closing of the nozzle 104, and therefore, the opening and closing is substantially performed between the sealing member 1043 and the needle valve 105.

In this case, the first gap G1 may be formed to have a size of 0.015 millimeters (mm) to 0.03 mm for the spraying of an aerosol with sufficiently small particles. For example, when the first gap G1 is smaller than 0.015 mm, it is difficult for a liquid composition to move along a narrow gap, and thus, a gas may be mainly sprayed, and some liquid droplets may be weakly sprayed as if it is boiling. On the other hand, when the first gap G1 is larger than 0.03 mm, large liquid droplets are mainly sprayed, which may cause an extremely large spraying amount per unit time, and the user may feel that the nasal cavity or oral cavity is wet. In addition, when the second gap G2 is formed too narrow due to a manufacturing tolerance of the sealing member 1043, a phenomenon which is the same phenomenon occurring due to the narrow first gap G1 may occur, and therefore, in consideration of this point, the second gap G2 may be formed sufficiently larger than the first gap G1. Accordingly, the inhaler 10 according to an embodiment may spray an aerosol with fine particles and easily adjust the spraying amount.

As described above, the inhaler 10 according to an embodiment may maintain a state in which the nozzle 104 is closed by receiving a force of the needle valve 105 normally pushing up in the first direction due to the preload of the spring 107. However, in the inhaler 10 according to an embodiment, when a suction force is applied through the mouthpiece 102 by the user, a negative pressure may be formed on the bottom of the piston 106, the piston 106 may move downward by overcoming the preload of the spring 107, and the needle valve 105 connected to the piston 106 may move downward, thereby forming the second gap G2 between the sealing member 1043 and the needle valve 105. Accordingly, the inhalable composition in the reservoir 103 may be discharged to the outside, for example, to the user's oral cavity through the first gap G1 by passing through the second gap G2 in the form of an aerosol. When the user stops inhaling, the piston 106 and the needle valve 105 may move upward again due to a restoring force of the spring 107 and the nozzle 104 may be closed to stop the spraying of the aerosol.

The lever 300 that facilitates the filling of the reservoir with the inhalable composition of the canister is applied to the inhaler 10 described above, and the specific configuration and operating mechanism of the lever 300 will be described below in detail with reference to FIGS. 5 and 6.

Referring to FIGS. 5 and 6, one side of the lever 300 of the inhaler 10 according to an embodiment may form a portion of the housing 101. Also, the other side thereof is movable between the bottom surface of the canister 200 and the second surface of the housing 101. When the one side of the lever 300 is pressed by a user, the other side of the lever 300 may move the canister 200 in a direction toward the reservoir 103. Accordingly, some of the inhalable composition in the canister 200 may be moved to and stored in the reservoir 103.

For example, the lever 300 may move in the first direction when pressed by the user. At this time, the canister 200 may move in the second direction by the lever 300.

For example, the reservoir 103 may be disposed adjacent to the first surface of the housing 101. The canister 200 may be disposed between the reservoir 103 and the second surface of the housing 101. That is, the canister 200 may be vertically disposed on the second surface of the housing 101, and at this time, the canister 200 may be disposed adjacent to a side surface opposite to the one side surface of the housing 101 formed by the lever 300. That is, the bottom surface of the canister 200 may be adjacent to the second surface of the housing 101, and the top of the canister 200 may be adjacent to the first surface of the housing 101.

Referring to FIG. 5, the lever 300 may include a first lever member 310 and a second lever member 320.

The first lever member 310 may form a portion of one side surface of the plurality of side surfaces of the housing 101.

The second lever member 320 may extend from the first lever member 310 toward the canister 200.

As shown in FIG. 6, when the first lever member 310 is pressed by the user and moves in the first direction, the second lever member 320 may move in the first direction together to be inserted between the bottom surface of the canister 200 and the second surface of the housing 101. Accordingly, the canister 200 may move in the second direction. That is, the second lever member 320 may push the canister 200 up toward the reservoir 103.

Referring to FIG. 6, the second lever member 320 may include a first inclined surface 3201. The first inclined surface 3201 may be formed in a shape in which a height decreases along the first direction. That is, a height of a portion close to the canister 200 may be smaller than a height of a portion close to the first lever member 310. When the first lever member 310 is pressed, the first inclined surface 3201 may be inserted between the bottom surface of the canister 200 and the second surface of the housing 101. At this time, the first inclined surface 3201 may come into contact with the bottom surface of the canister 200, and a corner portion of the bottom surface may slide on the first inclined surface 3201. Accordingly, the bottom surface of the canister 200 may be pushed up in the second direction. That is, when the first lever member 310 is pressed, the canister 200 may be moved in the second direction by the shape of the first inclined surface 3201.

Referring again to FIGS. 5 and 6, the lever 300 of the inhaler 10 according to an embodiment may further include a third lever member 330.

One end of the third lever member 330 may be fixed to the inside the housing, and the other end thereof may be coupled to the first lever member 310. In addition, the third lever member 330 may be positioned between the first lever member 310 and the second lever member 320. The third lever member 330 may be provided as, for example, a compression spring. As shown in FIG. 6, the third lever member 330 may be compressed in the first direction when the first lever member 310 is pressed. Also, when the first lever member 310 is not pressed, the third lever member 330 may be restored to return the second lever member 320 to its original position as shown in FIG. 5. As the second lever member 320 returns to its original position, the canister 200 may also return to its original position and the filling of the reservoir 103 with the inhalable composition may be stopped.

Specifically, the canister 200 of the inhaler 10 according to an embodiment may include an accommodation portion 210 and the injection hole 220.

The accommodation portion 210 may accommodate an inhalable composition.

One side of the injection hole 220 may be connected to the reservoir 103 and the other side thereof may be positioned in the accommodation portion 210. The injection hole 220 may operate to be opened or closed with respect to the reservoir 103. For example, the injection hole 220 may be opened when the first lever member 310 is pressed. When the injection hole 220 is opened, the inhalable composition accommodated in the accommodation portion 210 may be allowed to move to the reservoir 103. On the other hand, when the first lever member 310 is not pressed, the injection hole 220 may be closed. When the injection hole 220 is closed, a moving path of the inhalable composition to the reservoir 103 may be blocked, and accordingly, the filling of the reservoir 103 may be stopped.

Meanwhile, when the lever 300 is pressed in a state in which the inhalable composition in the reservoir 103 runs out, the reservoir 103 may be filled with the inhalable composition in an intake volume for 10 to 15 times of puffs. Also, in order to fill the reservoir 103 with the inhalable composition in an intake volume for 10 to 15 times of puffs, the lever 300 may need to be pressed for 3 to 5 seconds.

The operation of opening and closing the injection hole 220 may be controlled according to whether the user presses the lever 300.

As described above, when the first lever member 310 is pressed, the second lever member 320 may push up the bottom surface of the canister 200, that is, a bottom surface of the accommodation portion 210. At this time, as shown in FIG. 6, the accommodation portion 210 moves in the second direction, but one side of the injection hole 220 may be fixed to the reservoir 103. That is, the position of the injection hole 220 in the housing 101 does not change, but the position of the other side of the injection hole 220 in the accommodation portion 210 may change due to the rising of the accommodation portion 210 in the second direction.

For example, when the other side of the injection hole 220 is pressed deeply into the accommodation portion 210, the injection hole may be opened. Accordingly, the inhalable composition in the accommodation portion 210 may move to the reservoir 103 through the injection hole 220. At this time, the inhalable composition may move by a pressure difference between the reservoir 103 and the accommodation portion 210. That is, when the inhalable composition filled in the reservoir 103 by the user is consumed as the aerosol is discharged, the pressure in the reservoir 103 may be reduced, and therefore, when the injection hole 220 is opened, the inhalable composition may move from the accommodation portion 210 to the reservoir 103. On the other hand, when the reservoir 103 is sufficiently filled with the inhalable composition, the inhalable composition may not additionally move to the reservoir 103 even if the user presses the lever 300 to open the injection hole 220.

In addition, the canister 200 may further include a first elastic member (not shown) that is positioned in the accommodation portion 210 and compressed and restored according to a pressure applied to the injection hole 220. The first elastic member may be provided as, for example, a compression spring.

As described above, since one side of the injection hole 220 is fixed to the reservoir 103, the one side of the injection hole 220 may be pressed when the accommodation portion 210 receives an external force in the second direction by the lever 300. At this time, the first elastic member is compressed when the one side of the injection hole 220 is pressed, and thus, the accommodation portion 210 may move in the second direction with the injection hole 220 as an axis, as shown in FIG. 6. In addition, when the lever 300 is not pressed by the user and returns to its original position, the external force of the lever 300 relative to the canister 200 is also removed. At this time, the accommodation portion 210 may move in a direction opposite to the second direction as the first elastic member is restored. That is, if no external force is applied to the bottom surface of the accommodation portion 210 by the second lever member 320, the first elastic member may return the accommodation portion 210 to its original position as shown in FIG. 5.

In the inhaler 10 according to an embodiment, as the user presses the lever 300, the reservoir 103 may be easily filled with the inhalable composition accommodated in the canister 200.

The inhaler 10 according to an embodiment described above uses a method of filling the reservoir 103 with the inhalable composition accommodated in the canister 200, and thus, if a residual gas remains in the reservoir 103 during repeated refilling, it may be not easy to fill the reservoir 103 with the composition of the canister 200. Therefore, the relief valve 700 interlocked with the filling lever 300 may be applied to the inhaler 10 according to an embodiment to facilitate the filling of the reservoir 103, and the specific configuration and operating mechanism of the relief valve 700 will be described below in detail with reference to FIG. 7.

Referring to FIG. 7, the relief valve 700 of the inhaler 10 according to an embodiment may be provided on the reservoir 103 to be spaced apart from the one side of the canister 200. The relief valve 700 may be opened or closed to allow the reservoir 103 to communicate with the outside or block the reservoir 103.

The opening or closing operation of the relief valve 700 may be controlled by the lever 300 described above. For example, when the lever 300 is pressed by the user, the lever 300 may control the relief valve 700 so that the relief valve 700 is opened first and then the canister 200 is opened.

As described above, as the relief valve 700 interlocked with the lever 300 is applied to the inhaler 10 according to an embodiment, the residual gas in the reservoir 103 may be discharged and the reservoir 103 may be smoothly filled with the inhalable composition from the canister 200.

The lever 300 may be pressed by the user. At this time, the lever 300 may move toward the canister 200 to reach a first position, and when the lever 300 is further pressed by the user, the lever 300 may reach the second position. When the lever 300 is in the first position, the relief valve 700 may be opened, and when the lever 300 is in the second position, the relief valve 700 may be closed and the injection hole 220 of the canister 200 may be opened.

Specifically, the relief valve 700 may be interlocked with the lever 300 through the relief bar 800 and may be opened or closed by raising or lowering of the relief bar 800. The relief bar 800 may be disposed between the lever 300 and the canister 200. The relief bar 800 may have one end coupled to the relief valve 700 and the other end extending toward the lever 300. The relief bar 800 may open the relief valve 700 as the relief bar 800 moves in the second direction, and may close the relief valve 700 as the relief bar 800 returns to its original position.

The lever 300 may include a first support member 340 and the first protrusion member 350.

The first support member 340 may extend toward the canister 200.

The first protrusion member 350 may be rotatably coupled to the first support member 340 around a rotation shaft provided in the first support member 340. The first protrusion member 350 may have an end portion protruding in a radial direction from the rotation shaft at one side. The end portion of the first protrusion member 350 may be disposed in a standing state toward the first surface of the housing 101.

The other end of the relief bar 800 may be positioned between the first protrusion member 350 and the second surface of the housing 101. In addition, the end portion of the first protrusion member 350 may be inclined in a direction away from the canister 200 with respect to the rotation shaft.

When the lever 300 is pressed in the first direction, the first support member 340 may restrain the rotation of the first protrusion member 350. That is, the rotation of the first protrusion member 350 in the direction away from the canister 200 may be restrained in a state where the end portion stands up toward the first surface of the housing 101.

Therefore, when the lever 300 is pressed, the first protrusion member 350 also moves in the first direction to reach the first position, and at this time, the first protrusion member 350 may come into contact with the relief bar 800 and push the relief bar 800 up in the second direction without rotating.

As a result, when the lever 300 reaches the first position, the inhalable composition remaining in the reservoir 103 may be discharged to the outside.

Meanwhile, when the lever 300 returns to its original position, the first support member 340 may allow the rotation of the first protrusion member 350. That is, the rotation of the first protrusion member 350 in the direction toward the canister 200 may not be restrained in a state where the end portion stands up toward the first surface of the housing 101.

Accordingly, when the lever 300 moves from the second position or a position beyond the first position in a direction opposite to the first direction, the first protrusion member 350 may pass through the relief bar 800 and return to its original position without pushing the relief bar 800 up.

Referring back to FIG. 7, a distance between the first inclined surface 3201 of the second lever member 320 and the bottom surface of the canister 200 may be greater than a distance between the first protrusion member 350 and the relief bar 800. Therefore, when the lever 300 is moved in the first direction, the first protrusion member 350 may come into contact with the other end of the relief bar 800 first, before the first inclined surface 3201 comes into contact with the bottom surface of the canister 200. That is, the relief valve 700 provided on the reservoir 103 may be opened first, immediately before the lever 300 pushes the canister 200 to fill the reservoir 103.

Meanwhile, when the canister 200 is opened, that is, when the lever 300 reaches the second position and the first inclined surface 3201 pushes the canister 200 up in the second direction, the first protrusion member 350 may be positioned between the relief bar 800 and the canister 200.

Eventually, when the lever 300 reaches the second position, the reservoir 103 may be filled with the inhalable composition accommodated in the canister 200.

In addition, the injection hole 220 may be opened when the lever 300 is in the second position. When the injection hole 220 is opened, the inhalable composition accommodated in the accommodation portion 210 may be allowed to move to the reservoir. On the other hand, when the lever 300 is not in the second position, the injection hole 220 may be closed. When the injection hole 220 is closed, a moving path of the inhalable composition to the reservoir 103 may be blocked, and accordingly, the filling of the reservoir 103 may be stopped.

The operation of opening and closing the injection hole 220 may be controlled according to whether the user presses the lever 300.

In the inhaler 10 according to an embodiment described above, in order to prevent pressure of a residual gas remaining in the reservoir 103 from hindering a new composition from entering when the reservoir 103 is refilled with the composition using the filling lever 300, the relief valve 700 interlocked with the filling lever 300 may be provided to remove the residual gas in the reservoir 103 before the filling.

In addition, in the inhaler 10 according to an embodiment described above, the filling lever 300 may operate in two steps. In a first step, the relief valve 700 may be opened as a state before the filling, and in a second step, the relief valve 700 may be closed, thereby performing the filling.

In order to notify the time to replace the canister 200, the inhaler 10 according to an embodiment described above needs to deliver information on the remaining amount of the composition in the canister 200 to the user. Accordingly, in the inhaler 10 according to an embodiment, a counter function for notifying the available number of filling to the user by counting the number of filling of the filling lever 300 is applied. The counter 900 having such a function may calculate the number of filling in consideration of a total amount of the composition and a volume of the reservoir 103 and estimate the remaining number of filling by counting the number for each filling, and a counting method of mechanically operating in association with the operation of the filling lever 300 may be applied.

Hereinafter, the counter 900 will be described in more detail with reference to FIGS. 8 to 11.

Referring to FIG. 9, the counter 900 of the inhaler 10 according to an embodiment may be rotatably provided on one of the plurality of side surfaces of the housing 101. In this case, the counter 900 may be disposed between the canister 200 and the lever 300. The counter 900 may be rotatably provided in association with the lever 300. For example, the counter 900 may be rotated at a predetermined angle according to the operation of the lever 300 to notify the user of the remaining amount of the inhalable composition in the canister 200 by the color or number.

Specifically, the counter 900 may include a first counter member 910 and a second counter member 920.

The first counter member 910 may be formed in a circular shape and may have a center that is rotatably coupled to the inside of the housing 101. A symbol indicating the remaining amount of the canister 200 may be marked on one surface of the first counter member 910. For example, different colors or a series of numbers may be displayed on one surface of the first counter member 910 along a circumferential direction.

A plurality of second counter members 920 may be provided, and they may be formed in a sawtooth shape and arranged at regular intervals around the circumference of the first counter member 910.

The second counter members 920 may be engaged with the lever 300, and therefore, when the canister 200 is opened, the second counter members 920 may be moved in the circumferential direction and the first counter member 910 may be rotated.

At this time, the inhaler 10 according to an embodiment may further include a fastening portion 930 to prevent reverse rotation of the first counter member 910 other than the rotation by the lever 300.

The fastening portion 930 may have one end rotatably coupled to the housing 101 and the other end formed in a hook shape. The other end of the fastening portion 930 may be engaged with the second counter member 920. Accordingly, the fastening portion 930 may restrain the rotation of the first counter member 910 in one direction. That is, the fastening portion 930 may allow the rotation of the second counter member 920 in a direction toward the canister 200 by the lever 300, but may not allow the rotation of the second counter member 920 in a direction away from the canister 200.

As described above, when the lever 300 of the inhaler 10 according to an embodiment is pressed by the user, the canister 200 may be opened so that the reservoir 103 is filled with the inhalable composition. At this time, since the lever 300 and the counter 900 are interlocked with each other, the counter 900 may be rotated by the operation of the lever 300 to notify the remaining amount of the canister 200. When the lever 300 is pressed by the user, the lever 300 may be moved in the first direction and the lever 300 may move the canister 200 in the second direction and rotate the counter 900 at the same time.

Referring to FIGS. 9 and 10, the lever 300 may include a second support member 360 and a second protrusion member 370.

The second support member 360 may extend in the first direction toward the canister 200.

The second protrusion member 370 may be rotatably coupled to the second support member 360 around a rotation shaft provided in the second support member 360. The second protrusion member 370 may have an end portion formed to protrude in a radial direction from the rotation shaft at one side. The end portion of the second protrusion member 370 may be disposed in a standing state toward the first surface of the housing 101.

When the lever 300 is pressed, the second protrusion member 370 may be moved in the first direction and may apply a pressing force to one of the plurality of second counter members 920 in the first direction. Accordingly, the second counter member 920 may be rotated in a direction toward the canister 200, thereby rotating the first counter member 910.

As described above, when the lever 300 is pressed in the first direction, the second support member 360 may restrain the rotation of the second protrusion member 370. That is, the rotation of the second protrusion member 370 in a direction away from the canister 200 may be restrained in a state where the end portion stands toward the first surface of the housing 101.

Therefore, when the lever 300 is pressed, the second protrusion member 370 also comes into contact with the second counter member 920 as it moves in the first direction. At this time, the second protrusion member 370 may not be rotated but push the second counter member 920 to be rotated in a direction toward the canister 200.

As a result, when the lever 300 opens the canister 200, the counter 900 may be rotated to notify the changed remaining amount of the composition in the canister 200.

Meanwhile, when the lever 300 returns to its original position, the second support member 360 may allow the rotation of the second protrusion member 370. That is, the rotation of the second protrusion member 370 in the direction toward the canister 200 may not be restrained in a state where the end portion stands up toward the first surface of the housing 101.

Accordingly, when the lever 300 moves in a direction opposite to the first direction, the second protrusion member 370 may return to its original position without rotating the second counter member 920.

When the refilling of the composition in the reservoir 103 is repeated a certain number of times, the counter 900 may notify that there is no remaining composition in the canister 200, and the user who has confirmed it may remove the canister 200 from the housing 101.

Referring to FIG. 11, when the canister 200 is removed from the housing 101, the first counter member 910 may be rotated to return to its initial position. At this time, in order to return to the initial position, the counter 900 may rotate in a direction opposite to a rotation direction by the lever 300. To allow such reverse rotation, the inhaler 10 according to an embodiment may further include a reset portion 940.

The reset portion 940 may be disposed between the fastening portion 930 and the counter 900. The reset portion 940 may have one end rotatably mounted on the inside the housing 101 and the other end in contact with the fastening portion 930. When the canister 200 is removed from the housing 101, the reset portion 940 may push and rotate the fastening portion 930 in a direction toward the canister 200. Accordingly, the hook portion of the fastening portion 930 that is engaged with the second counter member 920 may be separated from the second counter member 920.

In addition, the counter 900 may further include a second elastic member (not shown) provided on the first counter member 910.

When the first counter member 910 is rotated by the lever 300, the second elastic member may be compressed along the rotation direction of the counter 900. When the rotational restriction does not act for the counter 900, for example, when the canister 200 is removed from the housing 101 to remove the rotational restriction by the fastening portion 930, the second elastic member may rotate the first counter member 910 to its original position.

Referring back to FIG. 3, the housing 101 of the inhaler 10 according to an embodiment may further include a display window, and the remaining amount in the canister 200 displayed on the counter 900 may be notified externally through the display window 110 so that the user may confirm it.

The display window 110 may be formed of a hole or a transparent material formed on one side of the housing 101. The display window 110 may be formed at a position corresponding to the first counter member 910 of the counter 900. The display window 110 may expose a color or number displayed by the first counter member 910 to the outside.

Meanwhile, in order to confirm the remaining amount of the composition in the reservoir 103, the reservoir 103 of the inhaler 10 according to an embodiment may be formed of a transparent material, and the housing 101 may further include the viewing window 120. The viewing window 120 may be a hole formed on one side of the housing 101 and may be formed at a position corresponding to the reservoir 103. This viewing window 120 may expose the amount of inhalable composition in the reservoir 103 to the outside. Accordingly, the user may easily check the remaining amount and whether the reservoir 103 is completely filled.

Since the counter 900 interlocked with the filling lever 300 is applied to the inhaler 10 according to an embodiment described above, the number of filling may be counted by using the vertical movement of the canister 200 when the reservoir 103 is filled with the inhalable composition from the canister 200, thereby notifying the remaining amount of the composition in the canister 200 and the information on the time to replace the canister 200.

As described above, the canister 200 may be mounted on the inhaler 10 according to an embodiment to be replaceable. Accordingly, when the inhalable composition in the canister 200 is used up, the user may replace the existing canister 200 with a new canister 200. For this, the cover 500 of the canister 200 is opened, however, when the cover 500 is opened in a state where the inhalable composition remains in the reservoir 103, the composition may be sprayed from the injection hole 220 of the canister 200 and push the canister 200 with a strong force. At this time, a problem that the canister 200 is discharged and a problem that the composition wets the user's hand may occur. To solve such problems, the cover lock 600 as a double locking device may be applied to the inhaler 10 according to an embodiment.

Referring to FIG. 12, the cover lock 600 of the inhaler 10 according to an embodiment may be positioned on the second surface of the housing 101. The cover lock 600 may be provided to be slidable, and may be moved to a position shown in FIG. 12A or 12B by the user.

For example, FIG. 12A shows the cover lock 600 in a locking position, and in the locking position, the cover lock 600 may restrict the rotation of the cover 500 so that the cover 500 is not opened. FIG. 12B shows the cover lock 600 moved to an unlocking position, and in the unlocking position, the cover lock 600 may allow the rotation of the cover 500 so that the cover 500 is opened. When the cover 500 is opened, the user may remove the canister 200 from the housing 101 of the inhaler 10.

Referring to FIGS. 13 and 14, the cover 500 may have one side rotatably coupled to the housing 101, and the other side detachably coupled to the housing 101 at a position corresponding to the bottom surface of the canister 200. The other side of the cover 500 may be separated from the housing 101 and the one side thereof may rotate to expose the bottom surface of the canister 200 to the outside. The rotation of the one side of the cover 500 may be controlled by the cover lock 600 as described above.

In addition, the cover lock 600 may first open the relief valve 700 before allowing the cover 500 to rotate.

Referring back to FIGS. 13 and 14, the relief valve 700 of the inhaler 10 according to an embodiment may be provided on the reservoir 103 to be spaced apart from the one side of the canister 200. This relief valve 700 may be opened or closed to allow the reservoir 103 to communicate with the outside or block the reservoir 103 from the outside.

Specifically, the relief valve 700 may be opened by the relief bar 800 that is raised when the cover lock 600 is in the unlocking position. The relief bar 800 may be positioned between the cover 500 and the cover lock 600.

The relief bar 800 may open the relief valve 700 as the relief bar 800 moves in the second direction, and may close the relief valve 700 as the relief bar 800 returns to its original position.

The movement of the relief bar 800 may be controlled by the cover lock 600.

As shown in FIG. 13, when the cover lock 600 is in the locking position, the relief bar 800 does not rise and the relief valve 700 may remain closed. On the other hand, as shown in FIG. 14, when the cover lock 600 is in the unlocking position, the relief bar 800 may be raised by the cover lock 600, and the relief valve 700 may be opened.

The cover lock 600 of the inhaler 10 according to an embodiment may include a first locking member 610 and a second locking member 620.

The first locking member 610 may slide on the second surface of the housing 101.

The second locking member 620 may extend from the first locking member 610 toward the first surface of the housing 101. The second locking member 620 may push up the other end of the relief bar 800 toward the first surface of the housing 101 when the first locking member 610 moves from the locking position to the unlocking position.

Specifically, one end of the second locking member 620 may be fixed to the first locking member 610, and the other end of the second locking member 620 may extend toward the first surface of the housing 101. A second inclined surface 6201 having a height decreasing toward the relief bar 800 may be formed on the other end of the second locking member 620.

When the first locking member 610 moves from the locking position to the unlocking position, the second inclined surface 6201 may gradually move the other end of the relief bar 800 toward the first surface of the housing 101. When the relief bar 800 rises toward the first surface of the housing 101 as described above, the relief valve 700 may be opened. In addition, when the first locking member 610 moves from the unlocking position to the locking position, the second locking member 620 including the second inclined surface 6201 may return to its original position and the relief bar 800 may move down toward the second surface of the housing 101, thereby closing the relief valve 700.

As a result, when the first locking member 610 moves from the locking position to the unlocking position, the relief valve 700 may be opened, the remaining composition in the reservoir 103 may be discharged, and the canister 200 may also be removed from the housing 101. Also, when the first locking member 610 is moved to the locking position again, the relief valve 700 may be closed.

Meanwhile, when the first locking member 610 is in the locking position, the second locking member 620 does not restrict the operation of the lever 300, but when the first locking member 610 is in the unlocking position, the second locking member 620 may restrict the operation of the lever 300.

As the relief valve 700 interlocked with the lever 300 is applied to the inhaler 10 according to an embodiment, the residual gas in the reservoir 103 may be discharged and the reservoir 103 may be smoothly filled with the inhalable composition from the canister 200.

However, the operation of the lever 300 with respect to the relief valve 700 may be allowed when the first locking member 610 of the cover lock 600 is in the locking position, as described above.

Referring to FIG. 14, when the first locking member 610 of the cover lock 600 is in the unlocking position, the second locking member 620 may block a path of the lever 300 moving in the first direction. For example, when the first locking member 610 is in the unlocking position, the second locking member 620 may be moved adjacent to the first protrusion member 350, thereby blocking the moving path of the first protrusion member 350. At the same time, the second locking member 620 may raise the relief bar 800. Accordingly, although the operation in which the lever 300 raises the relief bar 800 to open the relief valve 700, and then raises the canister 200 to open the injection hole 220 is not performed, the cover lock 600 may open only the relief valve 700 to discharge the remaining composition in the reservoir 103.

As described above, in the inhaler 10 according to an embodiment, the cover lock 600 may be applied as a double locking device to solve a problem that the canister cover 500 is easily opened so that the canister 200 mounted on the inside is discharged to the outside or the composition wets the user's hands. In addition, when the canister 200 is replaced, the inhaler 10 according to an embodiment may operate the relief valve 700 to discharge a medium residue in the reservoir 103.

In addition, in the inhaler 10 according to an embodiment, when the cover lock 600 is in the locking position, the cover 500 does not open and the filling lever 300 may normally operate, and when the cover lock 600 is in the unlocking position, only the cover 500 may be opened and the filling lever 300 may be caught by the second locking member 620 and may not operate. In addition, when the first locking member 610 is moved to the unlocking position, the inclined surface 6201 extending from the second locking member 620 may push the relief bar 800 up to open the relief valve 700 and discharge the residue in the reservoir.

## Claims

1. An inhaler (10) comprising:
a housing (101) having one surface, the other surface opposite to the one surface, and a plurality of side surfaces connecting the one surface and the other surface;
a mouthpiece (102) which is disposed on the one surface of the housing (101);
a reservoir (103) which communicates with the mouthpiece (102) and stores an inhalable composition; and
a canister (200) which accommodates the inhalable composition to be stored in the reservoir (103) and is connected to the reservoir (103) to be opened or closed,
wherein the canister (200) is detachably coupled to the reservoir (103) in the housing (101);
a lever (300) having one side forming a portion of the housing (101) and the other side being movable between a bottom surface of the canister (200) and the other surface of the housing (101),
wherein, when the lever (300) is pressed, the one side of the lever (300) moves in a first direction toward the canister (200) and the other side of the lever (300) presses the canister (200) to move in a second direction toward the reservoir (103);
**characterized by**
a relief valve (700) which is provided on the reservoir (103) to be spaced apart from the canister (200) and operates so that the reservoir (103) is opened or closed to the outside,
wherein the lever (300) moves in the first direction to reach a first position, and when the lever (300) is further pressed, the lever (300) moves in the first direction to reach a second position,
wherein when the lever (300) is in the first position, the relief valve (700) is opened, and when the lever (300) is in the second position, the relief valve (700) is closed, and the other side of the lever (300) presses the canister (200) to be opened.

2. The inhaler (10) of claim 1, further comprising:
a nozzle (104) which extends from the mouthpiece (102) to the reservoir (103); and
a needle valve (105) which is movably disposed inside the nozzle (104),
wherein, when a suction force is not applied to the mouthpiece (102), the needle valve (105) is maintained in a first state in which the nozzle (104) is closed, and
wherein, when a suction force is applied through the mouthpiece (102), the needle valve (105) is switched into a second state in which the nozzle (104) is opened.

3. The inhaler (10) of claim 2, further comprising:
a sealing member (1043) which is fixedly provided on one side of the nozzle (104),
wherein, in the first state, a first gap (G1) is formed between a front end of the needle valve (105) and the nozzle (104) and a lower end of the needle valve (105) comes into contact with the sealing member (1043), and
wherein, in the second state, a second gap (G2) is formed between the front end of the needle valve (105) and the sealing member (1043).

4. The inhaler (10) of claim 1, further comprising:
a counter (900) which is rotatably disposed between the canister (200) and the lever (300),
wherein the counter (900) is rotated at a predetermined angle according to the operation of the lever (300) to notify a remaining amount of the inhalable composition in the canister (200).

5. The inhaler (10) of claim 4, further comprising:
a cover (500) having one side rotatably coupled to the housing (101) and the other side detachably coupled to a position corresponding to the bottom surface of the canister (200); and
a cover lock (600) which is disposed adjacent to the one side of the cover (500) and to be slidable to a locking position restraining rotation of the cover (500) or an unlocking position allowing the rotation of the cover (500),
wherein the cover lock (600) opens the relief valve (700) in the unlocking position.

## Patentansprüche

1. Inhalationsvorrichtung (10), die Folgendes umfasst:
ein Gehäuse (101), das eine Fläche, eine andere Fläche, die der einen Fläche gegenüberliegt, und mehrere Seitenflächen, die die eine Fläche und die andere Fläche verbinden, aufweist;
ein Mundstück (102), das auf der einen Fläche des Gehäuses (101) angeordnet ist;
einen Vorratsbehälter (103), der mit dem Mundstück (102) in Verbindung steht und eine inhalierbare Zusammensetzung speichert; und
einen Kanister (200), der die inhalierbare Zusammensetzung, die im Vorratsbehälter (103) gespeichert werden soll, aufnimmt und so mit dem Vorratsbehälter (103) verbunden ist, dass er geöffnet oder geschlossen ist,
wobei der Kanister (200) mit dem Vorratsbehälter (103) im Gehäuse (101) lösbar gekoppelt ist;
einen Hebel (300), der eine Seite, die einen Abschnitt des Gehäuses (101) bildet, und die andere Seite, die zwischen einer Bodenfläche des Kanisters (200) und der anderen Fläche des Gehäuses (101) beweglich ist, aufweist,
wobei dann, wenn der Hebel (300) gedrückt wird, die eine Seite des Hebels (300) sich in einer ersten Richtung zum Kanister (200) bewegt und die andere Seite des Hebels (300) so auf den Kanister (200) drückt, dass er sich in einer zweiten Richtung zum Vorratsbehälter (103) bewegt;
**gekennzeichnet durch**
ein Entlastungsventil (700), das so auf dem Vorratsbehälter (103) vorgesehen ist, dass es vom Kanister (200) beabstandet ist, und derart arbeitet, dass der Vorratsbehälter (103) nach außen geöffnet oder verschlossen wird,
wobei sich der Hebel (300) so in der ersten Richtung bewegt, dass er eine erste Position erreicht, und dann, wenn der Hebel (300) weiter gedrückt wird, der Hebel (300) sich so in der ersten Richtung bewegt, dass er eine zweite Position erreicht,
wobei dann, wenn sich der Hebel (300) in der ersten Position befindet, das Entlastungsventil (700) geöffnet ist, und dann, wenn sich der Hebel (300) in der zweiten Position befindet, das Entlastungsventil (700) geschlossen ist und die andere Seite des Hebels (300) so auf den Kanister (200) drückt, dass er geöffnet wird.

2. Inhalationsvorrichtung (10) nach Anspruch 1, die ferner Folgendes umfasst:
eine Düse (104), die sich vom Mundstück (102) zum Vorratsbehälter (103) erstreckt; und
ein Nadelventil (105), das im Inneren der Düse (104) beweglich angeordnet ist,
wobei dann, wenn keine Saugkraft auf das Mundstück (102) ausgeübt wird, das Nadelventil (105) in einem ersten Zustand gehalten wird, in dem die Düse (104) geschlossen ist, und
wobei dann, wenn eine Saugkraft durch das Mundstück (102) ausgeübt wird, das Nadelventil (105) in einen zweiten Zustand umgeschaltet wird, in dem die Düse (104) geöffnet ist.

3. Inhalationsvorrichtung (10) nach Anspruch 2, die ferner Folgendes umfasst:
ein Dichtungselement (1043), das auf einer Seite der Düse (104) fest vorgesehen ist,
wobei im ersten Zustand ein erster Zwischenraum (G1) zwischen einem vorderen Ende des Nadelventils (105) und der Düse (104) gebildet wird und ein unteres Ende des Nadelventils (105) mit dem Dichtungselement (1043) in Kontakt gelangt, und
wobei im zweiten Zustand ein zweiter Zwischenraum (G2) zwischen dem vorderen Ende des Nadelventils (105) und dem Dichtungselement (1043) gebildet wird.

4. Inhalationsvorrichtung (10) nach Anspruch 1, die ferner Folgendes umfasst:
einen Zähler (900), der zwischen dem Kanister (200) und dem Hebel (300) drehbar angeordnet ist,
wobei der Zähler (900) gemäß der Betätigung des Hebels (300) um einen vorgegebenen Winkel gedreht wird, um eine Restmenge der inhalierbaren Zusammensetzung im Kanister (200) anzuzeigen.

5. Inhalationsvorrichtung (10) nach Anspruch 4, die ferner Folgendes umfasst:
eine Abdeckung (500), die eine Seite, die mit dem Gehäuse (101) drehbar gekoppelt ist, und die andere Seite, die an einer Position, die der Bodenfläche des Kanisters (200) entspricht, lösbar gekoppelt ist, aufweist; und
eine Abdeckungsverriegelung (600), die zu der einen Seite der Abdeckung (500) benachbart und so angeordnet ist, dass sie in eine Verriegelungsposition, die eine Drehung der Abdeckung (500) einschränkt, oder eine Entriegelungsposition, die die Drehung der Abdeckung (500) ermöglicht, verschiebbar ist.

## Revendications

1. Inhalateur (10) comportant :
un boîtier (101) ayant une première surface, la seconde surface étant opposée à la première surface, et une pluralité de surfaces latérales reliant la première surface et la seconde surface ;
un embout buccal (102) qui est disposé sur la première surface du boîtier (101) ;
un réservoir (103) qui communique avec l'embout buccal (102) et stocke une composition inhalable ; et
une cartouche (200) qui reçoit la composition inhalable à stocker dans le réservoir (103) et est reliée au réservoir (103) pour être ouvert ou fermé,
dans lequel la cartouche (200) est couplée au réservoir (103) de manière détachable dans le boîtier (101) ;
un levier (300) ayant un premier côté formant une partie du boîtier (101) et le second côté étant mobile entre une surface de fond de la cartouche (200) et la seconde surface du boîtier (101),
dans lequel, lorsque le levier (300) est pressé, le premier côté du levier (300) se déplace dans une première direction allant vers la cartouche (200) et le second côté du levier (300) presse la cartouche (200) pour se déplacer dans une seconde direction allant vers le réservoir (103) ;
**caractérisé par**
une soupape de décharge (700) qui est disposée sur le réservoir (103) de manière à être espacée de la cartouche (200) et fonctionne de sorte que le réservoir (103) est ouvert ou fermé par rapport à l'extérieur,
dans lequel le levier (300) se déplace dans la première direction pour atteindre une première position, et lorsque le levier (300) est encore pressé, le levier (300) se déplace dans la première direction pour atteindre une seconde position,
dans lequel, lorsque le levier (300) est dans la première position, la soupape de décharge (700) est ouverte, et lorsque le levier (300) est dans la seconde position, la soupape de décharge (700) est fermée, et le second côté du levier (300) presse sur la cartouche (200) pour l'ouvrir.

2. Inhalateur (10) selon la revendication 1, comportant en outre :
une buse (104) qui s'étend de l'embout buccal (102) au réservoir (103) ; et
une soupape à pointeau (105) qui est disposée de manière mobile à l'intérieur de la buse (104),
dans lequel, lorsqu'une force d'aspiration n'est pas appliquée à l'embout buccal (102), la soupape à pointeau (105) est maintenue dans un premier état dans lequel la buse (104) est fermée, et
dans lequel, lorsqu'une force d'aspiration est appliquée à travers l'embout buccal (102), la soupape à pointeau (105) est basculée dans un second état dans lequel la buse (104) est ouverte.

3. Inhalateur (10) selon la revendication 2, comportant en outre :
un élément d'étanchéité (1043) qui est disposé de manière fixe sur un côté de la buse (104),
dans lequel, dans le premier état, un premier espace (G1) est formé entre une extrémité avant de la soupape à pointeau (105) et la buse (104) et une extrémité inférieure de la soupape à pointeau (105) vient en contact avec l'élément d'étanchéité (1043), et
dans lequel, dans le second état, un second espace (G2) est formé entre l'extrémité avant de la soupape à pointeau (105) et l'élément d'étanchéité (1043).

4. Inhalateur (10) selon la revendication 1, comportant en outre :
un compteur (900) qui est disposé de manière rotative entre la cartouche (200) et le levier (300),
dans lequel le compteur (900) est tourné à un angle prédéterminé en fonction de l'actionnement du levier (300) pour notifier une quantité restante de la composition inhalable dans la cartouche (200).

5. Inhalateur (10) selon la revendication 4, comportant en outre :
un couvercle (500) ayant un premier côté couplé en rotation au boîtier (101) et le second côté couplé de manière détachable à une position correspondant à la surface de fond de la cartouche (200) ; et
un verrou de couvercle (600) qui est disposé au voisinage du premier côté du couvercle (500) et pour pouvoir coulisser jusqu'à une position de verrouillage restreignant une rotation du couvercle (500) ou une position de déverrouillage permettant la rotation du couvercle (500),
dans lequel le verrou de couvercle (600) ouvre la soupape de décharge (700) dans la position de déverrouillage.
